# EUROPEAN PATENT SPECIFICATION

(11) **EP 3 356 053 B1**
(45) Date of publication and mention of the grant of the patent: **10.11.2021**
(21) Application number: 16794427.1
(22) Date of filing: 30.09.2016
(51) Int. Cl.: B05B 11/00, A47K 17/00, A61L 2/22, A61L 2/26, E03D 9/00, A61L 9/14

(54) **ALL-PURPOSE DISPENSER FOR SPRAYING AND FUNNELLED DOSING OF LIQUIDS**
UNIVERSALSPENDER ZUM SPRÜHEN UND GETRICHTERTEN DOSIEREN VON FLÜSSIGKEITEN
DISTRIBUTEUR POLYVALENT POUR LA PULVÉRISATION ET LE DOSAGE EN ENTONNOIR DE LIQUIDES

(30) Priority: 02.10.2015 US 201514873227
(43) Date of publication of application: 08.08.2018
(73) Proprietor: Castillo Sancho, Fernando, 07800 Ibiza (Islas Baleares) (ES)
(72) Inventor: Castillo Sancho, Fernando, 07800 Ibiza (Islas Baleares) (ES)
(74) Representative: Lahidalga de Careaga, Jose Luis
(86) International application number: PCT/IB2016/055867
(87) International publication number: WO 2017/056050

(56) References cited:
- US-A- 3 589 563
- US-A- 4 235 373
- US-A- 5 249 718

## Description

### Subject of the invention

The invention refers to an all-purpose dispenser to spray and funnel the dosing of liquids which, with just one device, allows for the spraying of all types of liquids such as aromatic, medicinal and/or antiseptic or other essences, without the need for propellant gases or aerosols and which, simply by turning the nozzle attached to the liquid container, allows an auxiliary deposit to be filled which will funnel the liquid itself to direct uses such as purifying toilets or urinals.

### Field of application of the invention

The field of application of this invention is focused on the perfumery, hardware, cleaning articles and professional hygiene auxiliary industry sector.

### Prior art

Documents US4235373 discloses

A dispenser for spraying dosage of liquids without propellant gases or aerosols, based on a rectangular straight prism-shaped outside casing 19 with rounded edges, inside which there is a rectangular chassis-support to which there are elements attached.

The dispenser claims differs from this prior art in that the output nozzle comprises a perpendicular rotating lever in its middle so that the liquid can be sprayed from the front or back of the dispenser and turning the output nozzle using the lever to the opposite position releases the liquid through the back of the device which comprises a fuse and a deposit that collects the liquid, which rather than emerging as spray, does so under gravity along the channelling directly to the recipient to be cleaned or disinfected.

There are some antecedents for spray devices and resources.

One of the most important elements in spraying is the manner, time and method by which to vaporise the essences contained.

They do not normally operate continuously as that is neither necessary nor convenient, whether because of the essential effectiveness itself, or the excessive wear of continuous operation.

Some electrical and electronic models are familiar, which can be regulated to operate at intervals. Electricity or electronics are not convenient or adequate for some products and jobs.

There are also devices operated by photoelectric cells with the same problems as above, but better adapted to user requirements.

The prior art studied includes European patent EP 1450964 for "A Plug-in liquid spray" with the following main characteristics:
A plug-in liquid spray (10) comprising the following: a housing (12) with a generally flat vertical surface; a pair of pins (22) extending outward from said vertical surface to plug into a wall power takeoff providing alternating current; an impeller assembly (26) fitted in said housing and consisting of a piezoelectric activator (30) that expands and contracts in response to the fields of alternating current applied through the sides opposite it, and a spray plate (32) coupled to and made to vibrate by the expansion and contraction of said activator (30) to pulverise the liquid applied to one surface of said plate; a first electrical interconnection between one of said pins and one end of said piezoelectric activator, and a second interconnection between the other pin and an opposite end of that piezoelectric activator; an electronic switch (48) fitted linked to at least one of those first and second interconnections to control the application of voltages from said pins (22) to that piezoelectric activator (30); and an oscillator (54) connected to the electronic switch (48) to open and close said switch at high speed.

The inventor is also aware of the existence of European patent EP 0607182 for a "Liquid sprayer" with the following main characteristics:
The description is of an electrostatic liquid spray device consisting of a fuse-shaped nozzle (94) in contact with a container holding the liquid to be sprayed, with electrical power applied to the liquid at the nozzle discharge tip where the liquid emerges, principally under the influence of electrical forces inside the ligaments that break to form a spray of electrically charged liquid drops. The fuse is made of strips (10) in an open-cell deformable structure of polymeric foam material, and an edge of such material shaped to form a multiplicity of points (12) where they can be produced. A geared profile (12) is used with several teeth which create the points where the ligaments form.

The invention proposed clearly differs from the patents examined, pointing to the fact that these are electrical and not mechanical devices like the one in the proposed invention, and moreover do not allow the liquid to be channelled directly.

The device proposed in the invention is a somewhat complex mechanical device, albeit within a simplicity which resolves all the above problems and can to some degree be regulated to operate intermittently.

However, as reference to the current state of the art, it can be stated that at least the applicant is unaware of any other device which brings together the two functions, to spray and to funnel, with this invention's simplicity and reliability.

The invention proposed refers to an all-purpose dispenser to spray and funnel dosed liquids which makes it possible using a single device to spray all types of aromatic, medicinal and/or antiseptic essences without the need for propellant gases or aerosols and, by simply turning the liquid container nozzle, allows an auxiliary deposit to be filled which will funnel the liquid itself to direct functions to purify components which may be cleaned in this way, such as toilets, and comprising a straight, rectangular, prism-shaped external casing with rounded edges inside which a rectangular chassis-support is shown, moored to which are the remaining components of the device, comprising at least the following:
a liquid recipient with a pressure-difference-operated outlet nozzle at the top which, when pressed, sends part of the liquid to the exterior along the output channel;
said output nozzle has a perpendicular rotating lever in the middle so that the liquid can be discharged in spray form from the front or the back;
to press the nozzle for automatic operation and for the liquid to emerge at programmed intervals, provision is made for a device comprising a pressure trigger which has a substantially triangular element moored at one of its vertices on a rotation shaft while the upper vertex is secured by a tensor spring attached and secured at its opposite end;
welded to the middle part of the pressure trigger there is a cylindrical pressure cap exactly aligned with the top of the output nozzle and whose free end is carried by a load arm on an inertia flywheel, on one side of which there is a helical spiral, both elements integrated into an electric-motor-driven rotating shaft.

With this layout, the free end of the pressure trigger is supported on the helical spiral so that when the inertia flywheel turns and the pressure trigger slips on the end of the helical spiral because of the tension that element is under from the tensor spring, it is suddenly released and the output nozzle is pushed by the cylindrical pressure cap, allowing a certain quantity of disinfectant liquid to be sprayed automatically.

As the helical spiral rotates, it makes contact once more with the pressure trigger and the process begins again.

If the output nozzle is turned to the opposite position with the lever, the liquid is released through the rear of the device consisting of a fuse impregnated with liquid by capillarity action, releasing it gradually by evaporation and gravity into a deposit which collects the liquid and, rather than emerging in spray, it does so under gravity along the channelling, directly to the recipient to be cleaned or disinfected, e.g. a urinal or similar element.

In this very simple way, a single dispenser is able to spray liquid or funnel it as liquid to the component to be cleaned or disinfected.

### Description of the drawings

To complement this description and to aid in a better understanding of the characteristics of the invention, these descriptive specifications are accompanied by a page of plans forming an integral part hereof and which, by way of illustration and without limitation, show the following:
FIGURE 1.
   A view of a diagrammatic front cross-section of the inside of the dispenser.
FIGURE 2.
   A view of a diagrammatic side cross-section of the inside of the dispenser.

And in those figures, one reference is used to indicate identical elements, among which the following can be made out:
(1).- liquid recipient,
(2).- output nozzle,
(3).- rotation lever,
(4).- pressure trigger,
(5).- load arm,
(6).- tensor spring,
(7).- tensor spring mooring,
(8).- motor rotating shaft,
(9).- motor,
(10).- rotation vertex,
(11).- deposit,
(12).- helical spiral,
(13).- outer housing
(14).- chassis-support.
(15).- nozzle output funnel,
(16).- arm rotating shaft,
(17).- upper vertex,
(18).- cylindrical pressure cap,
(19).- inertia flywheel,
(20).- helical spiral shaft,
(21).- free end of the pressure trigger,
(22).- outer housing
(23).― fuse
(24).― channelling

### A preferred embodiment of the invention

The invention proposed as expressed in the heading to these descriptive specifications refers to an all-purpose spray and funnelled-dosage dispenser which makes it possible, with just one device, to spray all types of aromatic, medicinal and/or antiseptic essences, without the need for propellant gases or aerosols and which, simply by turning the liquid container nozzle, allows an auxiliary deposit to be filled which will funnel the liquid as such to direct functions for cleaning elements which can be purified in this way, such as toilets, and comprising principally the following elements:
Firstly there is a rectangular straight prism-shaped outer casing (22) with rounded edges inside which a rectangular chassis-support (14) is seen, to which the following elements are attached:
A liquid recipient (1) with a pressure-difference output nozzle (2) so that, when the nozzle (2) is pressed, a quantity of liquid is driven out along the output channel (15).

Said output nozzle (2) has a perpendicular rotation lever (3) in its middle so that liquid can be sprayed from the front or the back.

To press the nozzle (2) automatically and for the liquid to emerge at programmed intervals, provision is made for a device comprising a pressure trigger (4) which has a substantially triangular element moored at one of its vertices (10) on a rotation shaft (16) while the upper vertex (17) is secured by a tensor spring attached and secured at its opposite end (7).

Welded to the middle part of the pressure trigger (4) there is a cylindrical pressure cap (18) exactly aligned with the top of the output nozzle (2) and the free end (21) of the rotation lever (3) is carried by a load arm (5) comprising an inertia flywheel (19) on one side of which there is a helical spiral (12), both elements integrated into an electric-motor-driven (9) rotating shaft (20).

With this layout, the free end (21) of the pressure trigger (4) is supported on the helical spiral (12) so that when the inertia flywheel (19) turns and the pressure trigger slips on the end of the helical spiral (12) because of the tension that element is under from the tensor spring (6), it is released and the output nozzle (2) is pushed by the cylindrical pressure cap (18), allowing a certain quantity of liquid to be sprayed automatically.

As the helical spiral (12) rotates, it makes contact once more with the pressure trigger (4) and the process begins again.

If the output nozzle (2) is turned to the opposite position with the lever (3), the liquid is released through the rear of the device comprising a fuse (23) impregnated with liquid, releasing it gradually into a deposit (11) which collects the liquid and, rather than emerging in spray, it does so under gravity along the channelling (24), directly to the recipient to be cleaned or disinfected, such as a urinal or similar element.

In this very simple way, a single dispenser is able to spray liquid or funnel it as liquid to the component to be cleaned or disinfected.

Having sufficiently described the nature of the invention and its practical implementation, it must be recorded that the specifications indicated above and represented in the attached drawings may be modified in detail provided that this does not alter their main principles established in the previous paragraphs and summarised in the following claims.

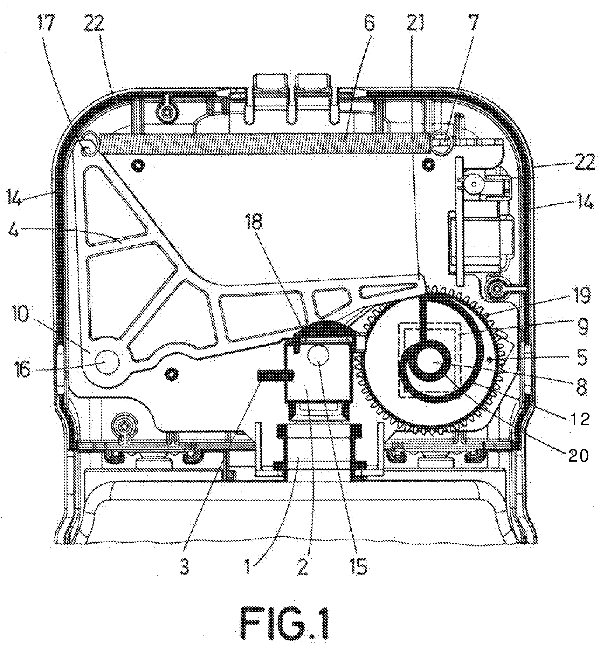

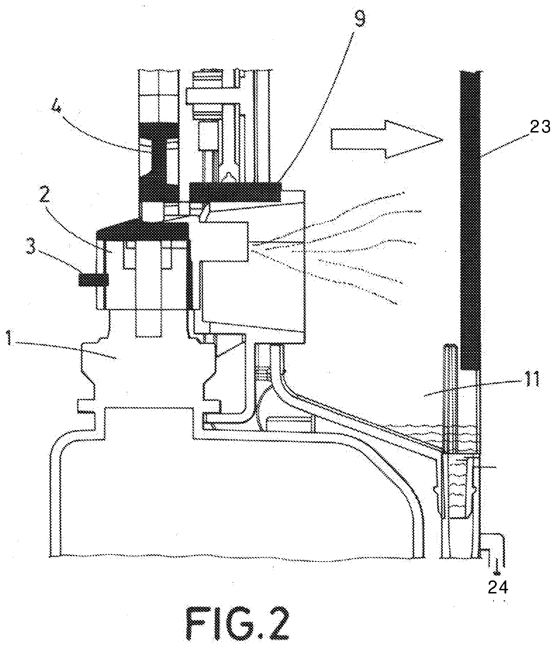

## Claims

1. An all-purpose dispenser for spraying and funneled dosage of liquids without propellant gases or aerosols, comprising a rectangular straight, prism-shaped outside casing (22) with rounded edges, inside which there is a rectangular chassis-support (14) to which the following elements are attached:
- a liquid recipient (1) with an output nozzle (2) at the top, the output nozzle (2) being provided with a spray output channel (15) and a perpendicular rotating lever (3) in its middle so that the liquid can be sprayed from the front or back of the all-purpose dispenser;
- a pressure trigger (4) comprising a substantially triangular part moored at one vertex (10) by a rotating shaft (16), an upper vertex (17) being secured by a tensor spring (6) moored and fixed at an opposite end (7);
wherein the all-purpose dispenser comprises a cylindrical pressure cap (18) welded to the middle of the pressure trigger (4) and exactly aligned with the top of the output nozzle (2), wherein a free end (21) of the pressure trigger (4) is supported on a load arm (5) consisting of an inertia flywheel (19), on one side of which there is a helical spiral (12), both the helical spiral (12) and the inertia flywheel (19) forming an integral part of an electric-motor-driven (9) rotating shaft (20);
and wherein, when the output nozzle (2) is turned to the opposite position using the lever (3), the liquid can be released through the back of the all-purpose dispenser which comprises a fuse (23) and a deposit (11) adapted to collect the liquid, which rather than emerging as spray, does so under gravity along a channelling (24) directly to a recipient to be cleaned or disinfected.

2. An all-purpose dispenser as set forth in Claim 1, whose output nozzle (2) at the top of the liquid recipient (1) is operated by pressure-difference so that, when the nozzle (2) is pressed, a quantity of liquid is impelled along the output channel (15).

3. An all-purpose dispenser as set forth in Claim 2, **characterized in that** the free end (21) of the pressure trigger (4) is supported on the helical spiral (12) so that, when the inertia flywheel (19) turns, the pressure trigger slips over the end of the helical spiral (12), where because of the tension the pressure trigger (4) is subjected to by the tensor spring (6), the pressure trigger (4) is suddenly released, pushing the output nozzle (2) by the cylindrical pressure cap (18) allowing a certain amount of liquid to be sprayed automatically, and when the helical spiral (12) comes once more as it turns into contact with the pressure trigger (4) the process begins again.

4. An all-purpose dispenser as set forth in Claim 3, **characterised in that** if the output nozzle (2) is turned to the opposite position using the lever (3), the liquid can be released through the rear of the all-purpose dispenser which comprises a fuse (23) impregnated with liquid and releasing the liquid gradually, collecting said liquid in the deposit (11) so that, rather than being sprayed, it emerges under gravity along the channelling (24) directly to the recipient to be cleaned or disinfected.

## Patentansprüche

1. Allzweckspender zum Versprühen und Dosieren von Flüssigkeiten ohne Treibgase oder Aerosole, **dadurch gekennzeichnet, dass** er aus einem rechteckigen geraden, prismenförmigen Außengehäuse (22) mit abgerundeten Kanten besteht, in dem sich ein rechteckiges Gehäuse befindet. Träger (14), an dem die folgenden Elemente befestigt sind:
- einen Flüssigkeitsbehälter (1) mit einer Austrittsdüse (2) so oben, mit einem Sprühaustrittskanal (15) und einem senkrechten Drehhebel (3) in seiner Mitte, damit die Flüssigkeit von vorne gesprüht werden kann oder Rückseite des Spenders;
- einen Druckauslöser (4) mit einem im Wesentlichen dreieckigen Teil, der an einem Scheitel (10) durch eine Drehwelle (16) verankert ist, wobei der obere Scheitel (17) durch eine Spannfeder (6) gesichert und am gegenüberliegenden Ende befestigt ist ( 7);
und mittig am Druckauslöser (4) angeschweißt ist eine zylindrische Druckkappe (18) genau fluchtend mit der Oberseite der Auslaufdüse (2) und mit dem freien Ende (21) des Druckauslösers (4) getragen von einem Lastarm (5), bestehend aus einem Schwungrad (19), an dessen einer Seite sich eine spiralförmige Spirale (12) befindet, wobei beide Elemente einen integralen Bestandteil einer elektromotorisch angetriebenen (9) rotierenden Welle bilden (20);
und wobei durch Drehen der Ausgabedüse (2) unter Verwendung des Hebels (3) in die entgegengesetzte Position die Flüssigkeit durch die Rückseite der Vorrichtung freigesetzt wird, die eine Sicherung und eine Ablagerung (11) umfasst, die die Flüssigkeit sammelt, die nicht als Sprühnebel austritt, tut dies unter Schwerkraft entlang der Kanalisierung direkt zum zu reinigenden oder zu desinfizierenden Behälter.

2. Allzweckspender nach Anspruch 1, dessen Ausgabedüse (2) an der Oberseite des Flüssigkeitsbehälters (1) durch Druckdifferenz betätigt wird, so dass, wenn die Düse (2) gedrückt wird, a Flüssigkeitsmenge wird entlang des Ausgangskanals (15) getrieben.

3. Allzweckspender nach Anspruch 2, **dadurch gekennzeichnet, dass** das freie Ende (21) des Druckauslösers (4) auf der Wendel (12) so abgestützt ist, dass bei Drehung des Schwungrades (19), der Druckauslöser rutscht aufgrund der Spannung der Spirale durch die Spannfeder (6) über das Ende der Wendel (12) und wird schlagartig gelöst, wobei die Auslaufdüse (2) durch die zylindrische Druckkappe (18 ) automatisches Versprühen einer bestimmten Flüssigkeitsmenge, und wenn die Wendel (12) beim Kontakt mit dem Druckauslöser (4) wieder auftaucht, beginnt der Vorgang von neuem.

4. Allzweckspender nach Anspruch 3, **dadurch gekennzeichnet, dass** wenn die Ausgabedüse (2) mit dem Hebel (3) in die entgegengesetzte Position gedreht wird, die Flüssigkeit durch die Rückseite der Vorrichtung abgegeben wird, die a . umfasst mit Flüssigkeit imprägniert und allmählich freigesetzt, wobei die Flüssigkeit in einem Behälter (11) gesammelt wird, so dass sie nicht versprüht wird, sondern unter Schwerkraft entlang der Kanäle direkt in den zu reinigenden oder zu desinfizierenden Behälter austritt.

## Revendications

1. Distributeur tout usage pour la pulvérisation et le dosage en entonnoir de liquides sans gaz propulseurs ni aérosols, **caractérisé en ce qu'**il est constitué d'un boîtier extérieur rectangulaire droit en forme de prisme (22) à bords arrondis, à l'intérieur duquel se trouve un châssis rectangulaire- support (14) auquel sont fixés les éléments suivants :
- un récipient à liquide (1) avec une buse de sortie (2) de sorte qu'en haut, avec un canal de sortie de pulvérisation (15) et un levier rotatif perpendiculaire (3) en son milieu afin que le liquide puisse être pulvérisé par l'avant ou à l'arrière du distributeur ;
- une gâchette à pression (4) comprenant une partie sensiblement triangulaire amarrée à un sommet (10) par un arbre tournant (16), le sommet supérieur (17) fixé par un ressort tenseur (6) amarré et fixé à l'extrémité opposée ( 7);
et ont été soudés au milieu de la gâchette de pression (4) il y a un capuchon de pression cylindrique (18) exactement aligné avec le haut de la buse de sortie (2), et avec l'extrémité libre (21) de la gâchette de pression (4) supporté sur un bras porteur (5) constitué d'un volant d'inertie (19), sur un côté duquel se trouve une spirale hélicoïdale (12), les deux éléments faisant partie intégrante d'un arbre rotatif (9) entraîné par un moteur électrique ( 20);
et où tourner la buse de sortie (2) à l'aide du levier (3) dans la position opposée libère le liquide par l'arrière du dispositif qui comprend un fusible et un dépôt (11) qui recueille le liquide, qui plutôt que d'émerger sous forme de pulvérisation, le fait par gravité le long de la canalisation directement jusqu'au récipient à nettoyer ou à désinfecter.

2. Distributeur universel selon la revendication 1, dont la buse de sortie (2) au sommet du récipient de liquide (1) est actionnée par différence de pression de sorte que, lorsque la buse (2) est enfoncée, une quantité de liquide est propulsée le long du canal de sortie (15).

3. Distributeur tout usage selon la revendication 2, **caractérisé en ce que** l'extrémité libre (21) de la gâchette à pression (4) prend appui sur la spirale hélicoïdale (12) de sorte que, lorsque le volant d'inertie (19) tourne , la gâchette de pression glisse sur l'extrémité de la spirale hélicoïdale (12) à cause de la tension à laquelle la spirale est soumise par le ressort tenseur (6) et est soudainement relâchée, poussant la buse de sortie (2) par le capuchon de pression cylindrique (18 ) permettant de pulvériser automatiquement une certaine quantité de liquide, et lorsque la spirale hélicoïdale (12) revient à nouveau en contact avec la gâchette de pression (4), le processus recommence.

4. Distributeur tout usage selon la revendication 3, **caractérisé en ce que** si la buse de sortie (2) est tournée dans la position opposée à l'aide du levier (3), le liquide est libéré par l'arrière du dispositif qui comprend un fusible imprégné de liquide et le libérant progressivement, recueillant ledit liquide dans un dépôt (11) de sorte qu'au lieu d'être pulvérisé, il ressorte par gravité le long de la canalisation, directement vers le récipient à nettoyer ou à désinfecter.
